# FASCICULE DE BREVET EUROPEEN

(11) **EP 2 968 813 B1**
(45) Date de publication et mention de la délivrance du brevet: **21.12.2016**
(21) Numéro de dépôt: 14709684.6
(22) Date de dépôt: 13.03.2014
(51) Int. Cl.: A61M 16/04, A61M 16/06, A61M 16/08, A61B 5/097, A61B 5/083

(54) **CANULE NASOPHARYNGÉE POUR CAPNOGRAPHIE EN FLUX SECONDAIRE**
NASOPHARYNGEALKANÜLE FÜR NEBENSTROM-KAPNOGRAFIE
NASOPHARYNGEAL CANNULA FOR SIDE-STREAM CAPNOGRAPHY

(30) Priorité: 15.03.2013 FR 1352333
(43) Date de publication de la demande: 20.01.2016
(73) Titulaire: Deltamedics, 60270 Gouvieux (FR)
(72) Inventeur: OZENNE, Jean-Christophe, 60270 Gouvieux (FR)
(74) Mandataire: Casalonga
(86) Numéro de dépôt international: PCT/EP2014/054942
(87) Numéro de publication internationale: WO 2014/140163

(56) Documents cités:
- EP-A1- 1 188 457
- WO-A1-2008/077033
- WO-A2-2004/103165
- DE-U1- 8 908 178
- US-A- 4 821 715
- US-A- 5 273 032
- US-A1- 2004 102 711
- US-A1- 2008 000 481
- US-A1- 2008 110 456
- US-A1- 2010 030 027
- US-A1- 2010 198 096
- US-A1- 2012 080 037

## Description

L'invention concerne un dispositif de libération des voies aériennes d'un patient, plus particulièrement une canule nasopharyngée permettant une injection de dioxygène ainsi qu'une mesure capnographique.

Une canule est un tube droit ou recourbé, souple ou rigide, permettant le passage d'un fluide tel que de l'air ou un liquide à travers un orifice. Une canule nasopharyngée est utilisée en médecine pour maintenir ouvertes les voies aériennes d'un patient inconscient, par exemple un patient sous anesthésie ou un patient plongé dans un coma, et plus particulièrement pour maintenir la perméabilité des voies respiratoires avec l'hypopharynx et pour faciliter l'élimination des sécrétions trachéobronchiques.

Une canule nasopharyngée se présente généralement sous la forme d'un tube souple en matière plastique comportant deux parties.

Une première partie est légèrement incurvée et souple, ou semi-rigide, de manière à suivre le trajet de la cavité nasale jusqu'au pharynx, et libérer ainsi un canal jusqu'à son pharynx. L'extrémité libre de la première partie vient se placer en arrière de la base de la langue, au-dessus de l'épiglotte. De cette manière, un passage d'air est maintenu vers les poumons.

Une seconde partie comprend une collerette qui repose sur l'entrée de la narine du patient une fois la canule en place et qui évite toute progression excessive de la canule.

Il existe plusieurs tailles de canule nasopharyngée. Les tailles varient entre l'enfant et l'adulte. Leur taille est exprimée principalement en fonction du diamètre interne de la canule.

Lors d'une opération sous anesthésie, un masque facial est généralement utilisé pour injecter du dioxygène au patient via le conduit de la canule nasopharyngée. L'utilisation d'un masque gêne cependant l'accès au visage du patient en cas de besoin.

De plus, l'utilisation d'un masque entraîne une mise en place complexe pour pouvoir réaliser une capnographie de l'air expiré par le patient.

La capnographie est une mesure de la concentration ou de la pression partielle de dioxyde de carbone dans l'air expiré par un patient. De telles mesures sont très utilisées sur des patients sous anesthésie. La présence de dioxyde de carbone dans l'air expiré sur plusieurs expirations par un patient venant d'être intubé permet notamment de confirmer que le tube endotrachéal est bien dans la trachée.

La capnographie permet par ailleurs d'obtenir une mesure indirecte de la pression partielle de dioxyde de carbone dans le sang artériel. Cette information permet d'évaluer l'état de vascularisation du patient. La capnographie reflète de manière directe la capacité d'élimination du dioxyde de carbone par les poumons du patient, et de manière indirecte, la production de dioxyde de carbone par les tissus et son transport jusqu'aux poumons.

Elle permet de détecter très tôt des signes de déficiences respiratoires telles qu'une hypoventilation, ou une déconnexion d'un circuit ou d'un tube dans l'oesophage. Lors d'une opération sous anesthésie, la capnographie permet de fournir des informations, telles que la fréquence et la régularité de ventilation, plus utiles que celles fournies par un oxymètre.

Elle fournit une méthode de détection rapide de conditions critiques, comme un tube trachéal mal positionné, un défaut de ventilation, un défaut circulatoire, et de prévenir des complications irréversibles.

Il est connu du document US 2007/0095347 un dispositif adapté pour réaliser à la fois une injection de dioxygène et un prélèvement des gaz expirés par le patient pour réaliser une mesure du taux de dioxyde de carbone en s'acquittant de l'utilisation d'un masque facial gênant.

Cependant, un tel dispositif n'utilise pas une canule nasopharyngée si bien que les voies respiratoires peuvent être obstruées, ce qui ne permet pas de garantir une injection d'oxygène optimale et un prélèvement de dioxyde de carbone dans les meilleures conditions.

Par ailleurs, le dispositif mis en oeuvre est, d'une part, lourd et très encombrant au niveau de la zone nasale du patient, et d'autre part, présente un harnachement complexe sur le patient.

Il est par ailleurs connu du document US 2008/0000481 un dispositif oropharyngé de forme adaptée pour être inséré dans la bouche d'un patient. Le dispositif comprend un corps comportant au moins deux canaux s'étendant dans le corps pour former deux passages d'air au travers desquels peut être injecté du dioxygène d'une part, et peut être extrait du dioxyde de carbone d'autre part. Les canaux sont formés dans le corps du dispositif qui comprend une collerette sur la portion proximale pour empêcher que le dispositif ne soit introduit trop profondément dans la bouche.

Ces dispositifs, uniquement oropharyngés, ne présentent pas de moyens de fixation simple et rapide sur le patient permettant son maintien sur le patient une fois le dispositif installé dans la bouche du patient, tout en permettant une extraction rapide du dispositif dans le cas où le patient se réveille ou dans un cas où une intubation est nécessaire.

Il est également connu des documents US 2007/267024, US 2008/308108, US 4 821 715, US 2012/080037, WO 2004/103165 et EP 1 188 457 un dispositif oro/nasopharyngé adapté pour former un passage de gaz à inhaler dans lequel est logée une paire de conduits adaptés pour être glissés dans le ou les passage(s) interne(s) pour injecter du dioxygène, sur une portion proximale notamment, et prélever du gaz expiré, sur une portion distale notamment, pour mesurer le taux de CO₂.

Ces dispositifs ne présentent pas non plus de moyens de fixation simple et rapide sur le patient permettant son maintien sur le patient une fois le dispositif installé dans la bouche ou le nez du patient, tout en permettant une extraction rapide du dispositif dans le cas où le patient se réveille ou dans un cas où une intubation est nécessaire.

Ces dispositifs présentent également l'inconvénient d'être en plusieurs parties, une partie composée de la canule et une partie composée des tubes d'injection et d'extraction à insérer. Le temps supplémentaire d'assemblage peut être critique en situation d'urgence notamment.

Une canule oro/nasopharyngée doit être installée et fixée sur le patient de sorte que celui-ci puisse la retirer au besoin, notamment dans le cas d'un réveil du patient. La fixation de la canule permet de maintenir en position le dispositif, mais cette fixation doit être amovible facilement.

Une canule nasopharyngée est le plus souvent mise en place durant la période de récupération post-anesthésie pour faciliter la toilette bronchique, ainsi que dans des cas où le patient est semi-conscient. Bien qu'une canule nasopharyngée soit généralement mieux supportée par un patient qu'une canule oropharyngée de type Guedel, certains patients peuvent ressentir une gêne plus ou moins prononcée pouvant entraîner un retrait de la canule nasopharyngée en urgence.

Il est aussi connu du document US 3 802 431 une canule nasale d'injection de dioxygène dans les narines du patient. Le dispositif se compose d'une petite canule placée à l'entrée de chaque narine pour injecter du dioxygène dans chaque narine à l'aide de tubes connectés aux canules et suffisamment souples pour être passés derrière les oreilles du patient une fois le dispositif en place sur le patient.

Cependant, un tel dispositif ne présente pas de canule nasopharyngée et ne permet que l'injection d'un fluide à l'entrée des narines.

L'invention vise à pallier les inconvénients mentionnés ci-dessus en fournissant une canule nasopharyngée adaptée pour être fixée sur le patient notamment à l'aide des tubes d'injection/extraction de la canule en place sur le patient.

Selon un aspect de l'invention, il est proposé une canule nasopharyngée comprenant un corps formé d'une portion tubulaire incurvée pour être insérée dans une narine d'un patient et d'une collerette apte à reposer sur la base de la narine du patient, la canule comprenant un conduit principal formant un passage de fluide entre la collerette et la portion tubulaire du corps, et deux conduits auxiliaires formés dans le corps et s'étendant de la collerette jusque dans le conduit principal.

La base de la narine du patient comprend la columelle et l'aile narinaire de la narine.

Selon une caractéristique générale de l'invention, chaque conduit auxiliaire débouche dans la collerette sur un orifice d'entrée/sortie orienté selon une direction radiale par rapport à l'axe du conduit principal de manière à être orienté latéralement de part et d'autre de la narine du patient lorsque la canule est installée sur le patient.

En orientant ainsi les orifices d'entrée/sortie, les tubes d'injection/extraction couplés aux orifices d'entrée/sortie s'étendent en direction des oreilles du patient, de chaque côté de la narine du patient. De cette manière chaque tube d'injection/extraction peut être passé derrière une oreille pour sécuriser la canule nasopharyngée sur le patient de manière facilement amovible.

De préférence, chaque orifice d'entrée/sortie est orienté selon une direction radiale par rapport à l'axe du conduit principal qui forme un angle compris entre -10° et 20°, et plus particulièrement entre 0° et 10°, avec un axe orthogonal au nez du patient lorsque la canule est installée sur le patient.

Un angle compris entre -10 et 20°, notamment un angle d'environ 0°, permet de diriger les tubes d'injection/extraction vers le rebord supérieur des oreilles, et ainsi faciliter le passage des tubes derrière les oreilles.

Selon un mode de réalisation, la collerette peut comprendre un premier et un second coudes d'entrée/sortie débouchant chacun sur un orifice d'entrée/sortie. Dans ce mode de réalisation, chaque conduit auxiliaire traverse la collerette jusque dans le coude d'entrée/sortie. La collerette peut comporter n'importe quelle épaisseur. Les coudes d'entrée/sortie sont en saillie à partir de la surface de la collerette.

Selon une alternative, chaque conduit auxiliaire peut comprendre une portion formée radialement dans la collerette, l'orifice d'entrée/sortie étant disposé radialement sur la collerette. Dans cette alternative, l'épaisseur de la collerette doit posséder une épaisseur suffisante pour permettre le passage des conduits auxiliaires.

De préférence, dans cette alternative, chaque orifice d'entrée/sortie disposé radialement sur la collerette est raccordé à un conduit auxiliaire via la portion de conduit auxiliaire s'étendant radialement dans la collerette en formant un angle compris entre 0° et 20° et plus particulièrement un angle d'environ 10° avec le plan défini par la surface de la collerette de manière à orienter l'orifice d'entrée/sortie correspondant vers le visage du patient lorsque la canule est installée sur le patient.

Cet angle permet d'orienter les tubes d'injection/extraction couplés aux orifices d'entrée/sortie de manière à les maintenir au plus près du visage du patient, et ainsi de réduire les risques qu'un élément ou un outil s'emmêle dans les tubes d'injection/extraction.

Avantageusement, la canule nasopharyngée peut comprendre deux tubes souples d'injection/extraction aptes à être passés derrière les oreilles du patient lorsque la canule est installée sur le patient, une première extrémité de chaque tube étant connectée à un orifice d'entré/sortie.

La connexion des tubes sur les orifices d'entrée/sortie peut être réalisée de manière amovible ou bien solidaire et inamovible. La présence des tubes solidaires sur la canule oropharyngée permet d'éviter un quelconque assemblage à part la connexion des extrémités libres des tubes à des moyens d'extraction, comme un capnographe, ou des moyens d'injection lors de l'installation de la canule sur le patient.

La canule nasopharyngée peut avantageusement comprendre une bague de serrage apte à maintenir les deux tubes ensemble et à coulisser le long des deux tubes de manière à maintenir la canule en place lorsqu'elle est installée sur le patient et que chaque tube passe derrière une oreille.

La bague de serrage permet, une fois les tubes passés derrière chaque oreille, de serrer le tube sous le menton en remontant la bague de serrage vers le menton. Cela permet d'assurer une fixation sécurisée de la canule et tout en permettant d'enlever rapidement la canule en cas de besoin.

Avantageusement, une seconde extrémité des tubes d'injection/extraction peut comprendre des moyens de connexion à des appareils d'injection ou d'extraction.

Les moyens de connexion peuvent être des connectiques spécifiques dédiées à des appareils de mesure ou d'injection de gaz. En ayant une connectique spécifique pour chaque tube, les risques d'erreur de branchement sont nuls, même en situation d'urgence, car chaque connectique est dédiée à un appareil spécifique.

De préférence, le premier conduit auxiliaire est destiné à l'injection de dioxygène dans les voies respiratoires du patient, et le second conduit auxiliaire est destiné à l'extraction de gaz expirés par le patient pour réaliser une mesure capnographique. Pour cela, le premier conduit auxiliaire comprend un orifice d'entrée connecté à un tube d'injection de dioxygène dans les voies respiratoires du patient, et le second conduit auxiliaire comprend un orifice de sortie connecté à un tube d'extraction de gaz expirés par le patient pour réaliser une mesure capnographique.

La portion tubulaire comprend de préférence une extrémité libre et une partie proximale, et le premier conduit auxiliaire s'étend jusqu'à une partie proximale de la portion tubulaire et le second conduit s'étend jusqu'à une partie distale comprenant l'extrémité libre de la portion tubulaire.

En séparant les lieux où débouchent les conduits auxiliaires dans le conduit principal, les perturbations liées notamment aux turbulences créées par l'injection de dioxygène sont réduites pour l'extraction des gaz expirés par le patient, et la mesure de dioxyde de carbone comprend un meilleur rapport signal sur bruit. D'après les mesures empiriques et la mécanique des fluides, il est préférable de réaliser une extraction des gaz expirés dans une zone plus distale que l'injection d'oxygène, de manière à être moins gêné par le flux d'oxygène.

Dans une variante, on peut également avoir deux tubes de prélèvement de dioxyde de carbone couplés aux deux orifices d'entrée sortie, les deux tubes étant couplés à un même dispositif de capnographie. De cette manière, le dispositif peut n'être utilisé que pour l'échantillonnage du dioxyde de carbone. Dans cette configuration, les deux conduits auxiliaires peuvent déboucher dans le conduit principal à des endroits similaires ou séparés. De manière similaire, la canule nasopharyngée peut être utilisée uniquement pour de l'injection de dioxygène via les deux conduits auxiliaires.

De préférence, le premier conduit auxiliaire est formé dans une paroi supérieure de la portion tubulaire du corps, et le second conduit auxiliaire est formé dans une paroi inférieure de la portion tubulaire du corps.

En réalisant le second conduit auxiliaire dans la paroi inférieure de la portion tubulaire, on réduit les risques d'occlusion par sécrétions, comme les glaires et la salive. En effet, lorsque le patient est allongé sur le dos, la surface inférieure de la paroi distale de la canule nasopharyngée installée dans le nez du patient jusqu'à son pharynx se trouve surélevée au niveau de l'extrémité libre par rapport au fond du pharynx où peuvent s'accumuler les sécrétions.

Au moins un des deux conduits auxiliaires peut avantageusement comprendre une section de forme oblongue au moins sur la portion tubulaire du corps.

De cette façon, le volume de fluide, gazeux notamment, pouvant être acheminé dans le conduit est plus important que dans le cas d'un conduit cylindrique en section. Les sections oblongues peuvent être réalisées de façon à suivre les contours du conduit principal de la canule nasopharyngée. De cette manière, on obtient des sections oblongues incurvées, ce qui permet de réaliser les conduits auxiliaires dans les parois de la canule sans avoir besoin d'augmenter de façon importante l'épaisseur des parois du corps de la canule. Et ce tout en conservant un volume de gaz transféré important.

Avantageusement, la canule peut comprendre au moins un conduit supplémentaire formé dans le corps et s'étendant depuis la collerette jusqu'à au moins la portion tubulaire du corps. Le conduit supplémentaire peut être destiné à l'aspiration de sécrétions dans le pharynx du patient de manière à éviter que les voies respiratoires ne soient obstruées par ces sécrétions. L'aspiration des sécrétions peut également être effectuée via un tube inséré dans le conduit principal.

Avantageusement, au moins un des conduits auxiliaires comprend un orifice débouchant dans le conduit principal possédant une forme tronconique avec une section plus grande que la section moyenne du conduit auxiliaire.

La forme tronconique de l'orifice débouchant sur le conduit principal au niveau de la portion tubulaire du corps permet de favoriser la bonne répartition du dioxygène injecté d'une part, et d'optimiser le prélèvement de dioxyde de carbone d'autre part.

De préférence, l'extrémité libre de la portion tubulaire du corps comprend une forme arrondie. La forme arrondie de l'extrémité libre permet de réduire les risques de blessure lors de l'introduction de la canule nasopharyngée, notamment par rapport à une canule nasopharyngée comportant une extrémité libre biseautée.

Le corps peut être réalisé en un matériau plastique souple et glissant. De cette façon, la canule nasopharyngée peut être introduite sans utiliser de gel lubrifiant.

D'autres avantages et caractéristiques de l'invention apparaîtront à l'examen de la description détaillée d'un mode de réalisation, nullement limitatif, et des dessins annexés, sur lesquels :
- la figure 1 représente, de manière schématique, une vue de dessous d'une canule nasopharyngée selon un mode de réalisation de l'invention ;
- la figure 2 illustre une vue en coupe selon un plan longitudinal II-II' de la canule nasopharyngée de la figure 1 ;
- la figure 3 illustre une vue en coupe selon un plan transversal III-III' de la canule nasopharyngée de la figure 1 ;
- la figure 4 illustre une vue en coupe selon un plan transversal IV-IV' de la canule nasopharyngée de la figure 1 ;
- la figure 5 illustre une vue en coupe selon un plan transversal V-V' de la canule nasopharyngée de la figure 1 ;
- la figure 6 illustre une vue en coupe selon un plan transversal VI-VI' de la canule nasopharyngée de la figure 1 ;
- la figure 7 présente un schéma d'une canule nasopharyngée de la figure 1 installée sur un patient.

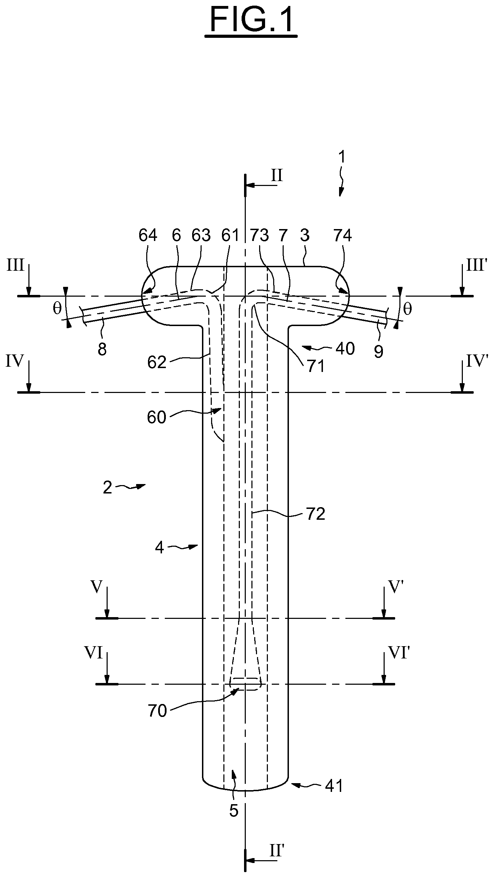

Sur la figure 1 est représentée schématiquement une vue de dessous d'une canule nasopharyngée 1 selon un mode de réalisation de l'invention.

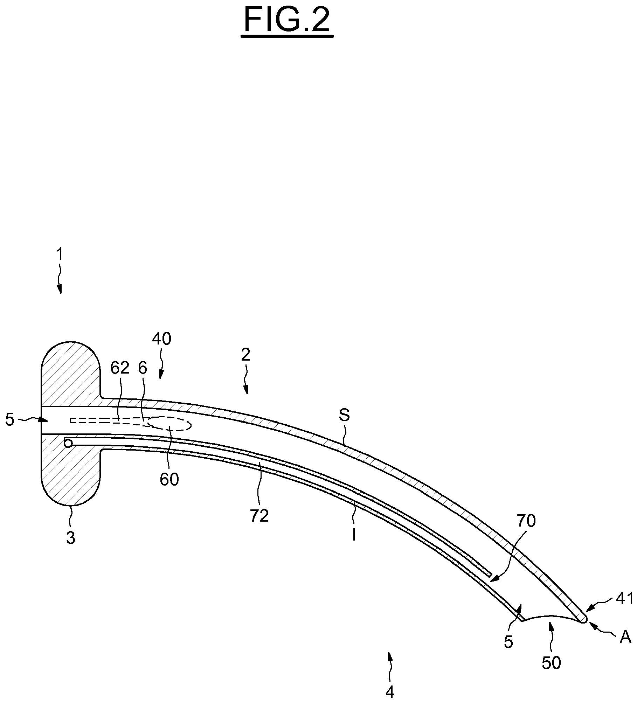

La canule nasopharyngée 1 comprend un corps 2 en matériau plastique souple glissant, comme par exemple du PVC médical, formé d'une collerette 3 et d'une portion tubulaire 4. Comme cela est illustré sur la figure 2 présentant une coupe longitudinale selon le plan II-II' de la canule 1 de la figure 1, la portion tubulaire 4 possède une forme incurvée de manière à permettre l'insertion de la canule nasopharyngée 1, et notamment sa portion tubulaire 4, dans la narine d'un patient jusque dans son pharynx de manière à passer derrière la base de la langue et ainsi maintenir le pharynx ouvert.

La collerette 3 est destinée à reposer sur l'extérieur de la base de la narine du patient une fois la canule 1 insérée dans la narine du patient. Le corps 2 comprend un conduit principal 5 s'étendant de la collerette 3 jusqu'à l'extrémité libre 41 de la portion tubulaire 4. Le conduit principal 5 forme ainsi un passage fluidique entre la collerette 3 et la portion tubulaire 4 du corps 2 de la canule nasopharyngée 1. Une fois la canule 1 installée sur le patient, le conduit principal 5 forme un passage fluidique entre le pharynx et la narine du patient.

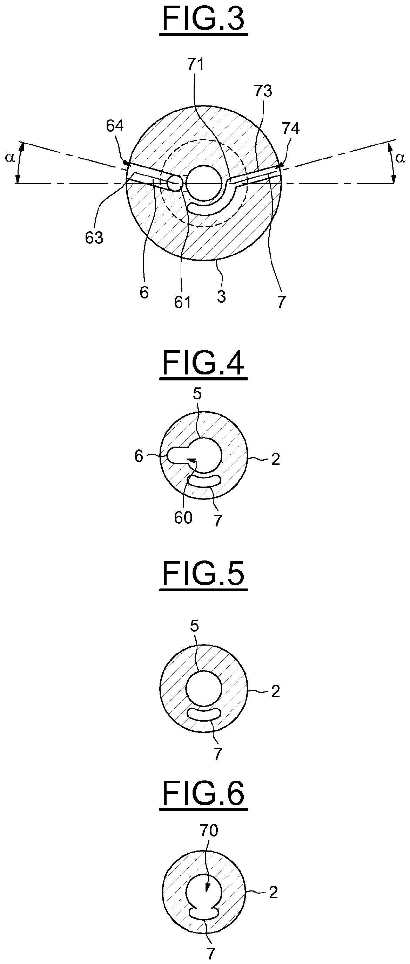

Comme cela est illustré sur les figures 1 et 2, la canule nasopharyngée 1 comprend un premier conduit auxiliaire 6 formé dans l'épaisseur du corps 2 séparant le conduit principal 5 de l'extérieur du corps 2. Le premier conduit auxiliaire 6 s'étend de la collerette 3 jusqu'à une partie proximale 40 de la portion tubulaire 4. Il débouche dans le conduit principal 5 via une première ouverture 60, comme cela est représenté sur les figures 1 et 3 ainsi que sur la figure 4 qui représente une vue selon le plan de coupe IV-IV' de la canule nasopharyngée 1 de la figure 1.

Comme représenté sur la figure 1 et sur la figure 3 qui représente une vue en coupe selon le plan III-III' de la canule nasopharyngée 1 de la figure 1, le premier conduit auxiliaire 6 comprend un coude 61 joignant une première portion 62 du premier conduit auxiliaire 6 s'étendant parallèlement à l'axe longitudinale II-II' jusqu'à la première ouverture 60 et une seconde portion 63 du premier conduit auxiliaire 6 s'étendant dans la collerette 3. La seconde portion 63 s'étend dans la collerette 3 selon une direction radiale par rapport à l'axe longitudinal II-II', comparable à l'axe du conduit principal 5, jusqu'à un orifice d'entrée 64.

La canule nasopharyngée 1 comprend un second conduit auxiliaire 7 également formé dans l'épaisseur du corps 2 et ayant une forme oblongue incurvée en section. Le second conduit auxiliaire 7 traverse le corps 2 depuis la collerette 3 et débouche dans le conduit principal 5 via une seconde ouverture 70 disposée en amont de l'extrémité libre 41 de la portion tubulaire 4, comme représenté sur les figures 1 et 2 ainsi que sur la figure 6 qui représente une vue selon le plan de coupe VI-VI' de la canule nasopharyngée 1 de la figure 1. En positionnant ainsi la seconde ouverture 70 en amont de l'extrémité libre 41 de la portion tubulaire, on réduit les risques d'obstruction de la seconde ouverture 70 par des sécrétions dans la gorge.

Ces risques sont encore plus limités de par le choix de réaliser le second conduit auxiliaire 70 dans la paroi inférieure *I* de la portion tubulaire 4. En effet, lorsque le patient est allongé sur le dos la paroi inférieure *I* de la portion tubulaire 4 de la canule nasopharyngée 1 installée dans le nez du patient se trouve surélevée au niveau de l'extrémité libre 41 par rapport au fond du pharynx où peuvent s'accumuler les sécrétions.

Pour réduire d'autant plus l'éventuelle accumulation de sécrétions dans le pharynx, la canule nasopharyngée 1 peut comprendre un conduit supplémentaire, non représenté, formé dans le corps 2 et s'étendant de la collerette 3 à l'extrémité libre 41 de la portion tubulaire 4. Le conduit supplémentaire est préférentiellement réalisé dans la paroi supérieure S de la portion tubulaire 4 de manière à être en contact avec le fond du pharynx où s'accumulent les sécrétions. Le conduit supplémentaire est couplé à des moyens d'aspiration de manière à éliminer les sécrétions.

Comme représenté sur les figures 1 à 3, le second conduit auxiliaire 7 comprend un coude 71 joignant une première portion 72 du second conduit auxiliaire 7 s'étendant selon l'axe longitudinal II-II' jusqu'à la seconde ouverture 70 et une seconde portion 73 du second conduit auxiliaire 7 s'étendant dans la collerette 3. La seconde portion 73 du second conduit auxiliaire 7 s'étend dans la collerette 3 selon une direction radiale par rapport à l'axe longitudinal II-II' jusqu'à un orifice de sortie 74.

La paroi supérieure S de la portion tubulaire 4 possède une forme arrondie *A* à l'extrémité libre 41. La forme arrondie *A* permet de réduire les risques de blessure lors de l'introduction de la canule nasopharyngée 1 dans la narine du patient.

Pour optimiser l'injection ou l'extraction d'un fluide via un conduit auxiliaire 6 ou 7, le premier conduit auxiliaire 6 et le second conduit auxiliaire 7 possèdent chacun une forme tronconique à l'extrémité débouchant sur la première ou la seconde ouverture 60 ou 70. Les sections de la première ouverture 60 et de la seconde ouverture 70 sont ainsi respectivement plus grandes que les sections des premier et second conduits auxiliaires 6 et 7. Cette forme tronconique permet d'optimiser le prélèvement dans le cas d'une extraction ou de favoriser la répartition du fluide injecté dans le conduit principal 5.

Le premier conduit auxiliaire 6 est destiné à délivrer un flux de dioxygène. Le second conduit auxiliaire 7 est destiné à prélever une portion des gaz expirés par le patient pour mesurer le taux de dioxyde de carbone dans les gaz expirés grâce à un capnographe.

Pour cela, l'orifice d'entrée 64 et l'orifice de sortie 74 sont respectivement connectés à un tube d'injection 8 et un tube d'extraction 9 comme cela est illustré sur la figure 1. La connexion est réalisée par soudure ou par surmoulage de la collerette 3 sur les tubes d'injection et d'extraction 8 et 9, ou encore à l'aide de connectique.

L'extrémité libre du tube d'injection 8 comprend une connectique spécifique par exemple un raccord conique standard pour dioxygène, destinée à être couplée à un dispositif délivrant un flux de dioxygène, optionnellement via un réducteur de section de tube. L'extrémité libre du tube d'injection 9 comprend en revanche une connectique spécifique, par exemple un raccord de type « luer lock », destinée à être couplée à un capnographe.

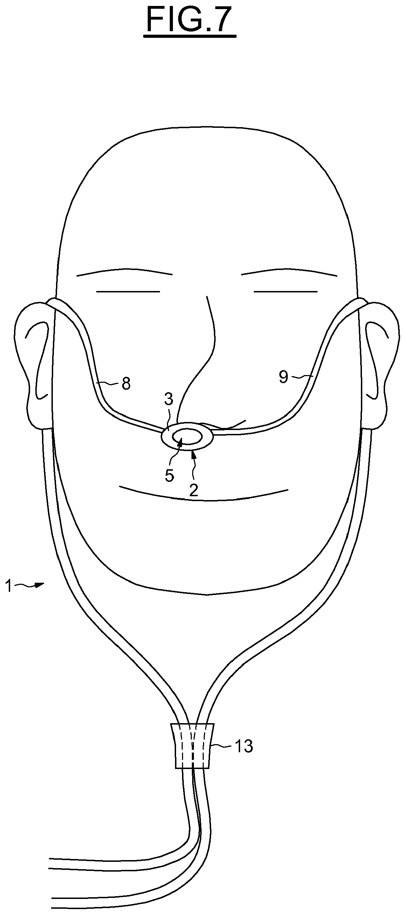

Comme cela est illustré sur la figure 3, la seconde portion 63 du premier conduit auxiliaire 6 et la seconde portion 73 du second conduit auxiliaire 7 forment chacune un angle α avec un axe passant par le premier coude 61 et le second coude 62. L'angle α possède une valeur comprise entre -10° et 20° et préférentiellement avoisinant 0°. Cet angle permet au tube d'injection 8 et au tube d'extraction 9 d'être chacun orientés en direction du rebord supérieur d'une oreille du patient de manière à faciliter le passage des tubes d'injection et d'extraction 8 et 9 derrière les oreilles, comme cela est illustré sur la figure 7.

De plus la seconde portion 63 du premier conduit auxiliaire 6 et la seconde portion 73 du second conduit auxiliaire 7 peuvent former chacune un angle compris entre 0° et 20° et plus particulièrement d'environ 10° avec le plan défini par la surface de la collerette 3 de manière à orienter les tubes d'injection et d'extraction 8 et 9 vers le visage du patient et optimiser ainsi le maintien en position de la canule nasopharyngée 1. Les tubes d'injection et d'extraction 8 et 9 étant amenés ainsi au près du visage du patient, les risques qu'un élément ou un outil s'emmêle dans les tubes sont en outre réduits.

La figure 7 présente un schéma d'une canule nasopharyngée 1 installée sur un patient selon le mode de réalisation de la figure 1. Le passage des tubes d'injection et d'extraction 8 et 9 derrière les oreilles du patient permet de maintenir la canule nasopharyngée 1 en place sur la narine du patient. Dans ce mode de réalisation, la canule nasopharyngée 1 comprend en plus une bague de serrage 13 couplée aux tubes d'injection et d'extraction 8 et 9. La bague de serrage 13 est montée coulissante le long des tubes d'injection et d'extraction 8 et 9 de manière à permettre de remonter la bague 13 jusqu'à ce que les tubes d'injection et d'extraction soient serrés sous le menton du patient de sorte que la canule nasopharyngée 1 soit maintenue de manière sécurisée en position sur le patient.

De manière à améliorer l'évacuation de l'humidité présente dans le tube d'extraction 9, le tube d'extraction peut comprendre une portion de quelques centimètres, 5 cm par exemple, de tube en Nafion. Cette portion est de préférence située à 2 cm de la collerette 3.

Dans une alternative, le premier conduit auxiliaire 6 formé dans l'épaisseur du corps 2 peut comporter une section de forme oblongue incurvée en section similaire à la section du second conduit auxiliaire 7. Dans le cas où les deux conduits auxiliaires 6 et 7 possèdent une forme oblongue incurvée en section, les deux conduits peuvent s'étendre dans la portion tubulaire 4 de manière à être opposés symétriquement à l'axe du conduit principal 5. Ainsi, par exemple, si le second conduit auxiliaire 7 est réalisé dans la paroi inférieure *I* de la portion tubulaire 4, le premier conduit auxiliaire 6 peut être réalisé dans la paroi supérieure S de la portion tubulaire.

L'invention proposée fournit une canule nasopharyngée apte à être installée et fixée rapidement sur le patient de manière amovible tout en étant maintenue de manière sécurisée en position sur le patient. De plus, la canule nasopharyngée proposée est réalisée en une seule pièce ou est préassemblée de manière à ce qu'il n'y ait plus qu'à connecter les tubes d'injection/extraction aux appareils destinés. Cela permet de réduire le temps d'installation et les risques d'erreur de branchement.

## Revendications

1. Canule nasopharyngée (1) comprenant un corps (2) formé d'une portion tubulaire (4) incurvée pour être insérée dans une narine d'un patient et d'une collerette (3) apte à reposer sur la base de la narine du patient, la canule (1) comprenant un conduit principal (5) formant un passage de fluide entre la collerette (3) et la portion tubulaire (4) du corps (2), et deux conduits auxiliaires (6, 7) formés dans le corps (2) et s'étendant de la collerette (3) jusque dans le conduit principal (5), **caractérisée en ce que** chaque conduit auxiliaire (6, 7) débouche dans la collerette (3) sur un orifice d'entrée/sortie (64, 74) orienté selon une direction radiale par rapport à l'axe (II-II') du conduit principal (5) de manière à être orienté latéralement de part et d'autre de la narine du patient lorsque la canule (1) est installée sur le patient.

2. Canule (1) selon la revendication 1, dans laquelle chaque orifice d'entrée/sortie (64, 74) est orienté selon une direction radiale par rapport à l'axe (II-II') du conduit principal (5) qui forme un angle compris entre -10° et 20°, et plus particulièrement entre 0° et 10°, avec un axe orthogonal à l'axe du nez du patient lorsque la canule (1) est installée sur le patient, de manière à orienter les orifices d'entrée/sortie (64, 74) en direction du rebord supérieur des oreilles du patient lorsque la canule (1) est installée sur le patient.

3. Canule (1) selon l'une des revendications 1 ou 2, dans laquelle la collerette (3) comprend pour chaque conduit auxiliaire (6, 7) un coude d'entrée/sortie, chaque conduit auxiliaire (6, 7) traversant la collerette (3) jusque dans le coude d'entrée/sortie débouchant sur l'orifice d'entrée/sortie (64, 74).

4. Canule (1) selon l'une des revendications 1 ou 2, dans laquelle chaque conduit auxiliaire (6, 7) comprend une portion (63, 73) formée radialement dans la collerette (3), l'orifice d'entrée/sortie (64, 74) étant disposé radialement sur la collerette (3).

5. Canule (1) selon la revendication 4, dans laquelle chaque orifice d'entrée/sortie (64, 74) disposé radialement sur la collerette (3) est raccordé à un conduit auxiliaire (6, 7) via la portion (63, 73) de conduit auxiliaire (6, 7) s'étendant radialement dans la collerette (3) en formant un angle compris entre 0° et 20° et plus particulièrement un angle d'environ 10° avec le plan défini par la surface de la collerette (3) de manière à orienter l'orifice d'entrée/sortie correspondant (64, 74) vers le visage du patient, lorsque la canule (1) est installée sur le patient.

6. Canule (1) selon l'une des revendications 1 à 5, comprenant deux tubes souples d'injection/extraction (8, 9) aptes à être passés derrière les oreilles du patient lorsque la canule (1) est installée sur le patient, une première extrémité de chaque tube étant connectée à un orifice d'entré/sortie (64, 74).

7. Canule (1) selon la revendication 6, comprenant une bague de serrage apte à maintenir les deux tubes (8, 9) ensemble et à coulisser le long des deux tubes (8, 9) de manière à maintenir la canule (1) en place lorsqu'elle est installée sur le patient et que chaque tube (8, 9) passe derrière une oreille.

8. Canule (1) selon l'une des revendications 6 ou 7, dans laquelle une seconde extrémité des tubes d'injection/extraction (8, 9) comprend des moyens de connexion à des appareils d'injection ou d'extraction.

9. Canule (1) selon l'une des revendications 1 à 8, dans laquelle le premier conduit auxiliaire (6) comprend un orifice d'entrée (64) connecté à un tube d'injection (8) de dioxygène dans les voies respiratoires du patient, et le second conduit auxiliaire (7) comprend un orifice de sortie (74) connecté à un tube d'extraction (9) de gaz expirés par le patient pour réaliser une mesure capnographique.

10. Canule (1) selon l'une des revendications 1 à 9, dans laquelle le premier conduit auxiliaire (6) s'étend jusqu'à une partie proximale (40) de la portion tubulaire et le second conduit (7) s'étend jusqu'à une partie distale comprenant l'extrémité libre (41) de la portion tubulaire (4).

11. Canule (1) selon l'une des revendications 1 à 10, dans laquelle le premier conduit auxiliaire (6) est formé dans une paroi supérieure (S) de la portion tubulaire (4) du corps (2), et le second conduit auxiliaire (7) est formé dans une paroi inférieure (I) de la portion tubulaire (4) du corps (2).

12. Canule (1) selon l'une des revendications 1 à 11, dans laquelle au moins un des conduits auxiliaires (6, 7) comprend une section de forme oblongue au moins sur la portion tubulaire (4) du corps (2).

13. Canule (1) selon l'une des revendications 1 à 12, dans laquelle au moins un des conduits auxiliaires (6, 7) comprend un orifice débouchant dans le conduit principal (5) possédant une forme tronconique avec une section plus grande que la section moyenne du conduit auxiliaire (6, 7).

14. Canule (1) selon l'une des revendications 1 à 13, dans laquelle l'extrémité libre (41) de la portion tubulaire (4) du corps (2) est dotée d'une forme arrondie.

15. Canule (1) selon l'une des revendications 1 à 14, dans laquelle le corps (2) est réalisé en un matériau plastique souple et glissant.

## Patentansprüche

1. Nasopharyngealkanüle (1), umfassend einen Körper (2), der aus einem röhrenförmigen Abschnitt (4), der gebogen ist, so dass er in ein Nasenloch eines Patienten eingeführt werden kann, und einem Bund (3) gebildet ist, der auf der Basis des Nasenlochs des Patienten ruhen kann, wobei die Kanüle (1) einen Hauptkanal (5), der einen Fluiddurchgang zwischen dem Bund (3) und dem röhrenförmigen Abschnitt (4) des Körpers (2) bildet, und zwei Hilfskanäle (6, 7) umfasst, die im Körper (2) ausgebildet sind und sich vom Bund (3) bis in den Hauptkanal (5) erstrecken, **dadurch gekennzeichnet, dass** jeder Hilfskanal (6, 7) an einer Eingangs-/Ausgangsöffnung (64, 74), die bezüglich der Achse (II - II') des Hauptkanals (5) radial ausgerichtet ist, in den Bund (3) mündet, so dass sie seitlich jeder Seite des Nasenlochs des Patienten ausgerichtet ist, wenn die Kanüle (1) an den Patienten angelegt ist.

2. Kanüle (1) nach Anspruch 1, wobei jede Eingangs-/Ausgangsöffnung (64, 74) in einer bezüglich der Achse (II - II') des Hauptkanals (5) radialen Richtung ausgerichtet ist, der einen Winkel von zwischen -10° und 20°, und insbesondere zwischen 0° und 10°, mit einer orthogonal zu der Achse der Nase des Patienten verlaufenden Achse bildet, wenn die Kanüle (1) an den Patienten angelegt ist, um die Eingangs-/Ausgangsöffnungen (64, 74) in der Richtung des oberen Rands der Ohren des Patienten auszurichten, wenn die Kanüle (1) an den Patienten angelegt ist.

3. Kanüle (1) nach Anspruch 1 oder 2, wobei der Bund (3) eine Eingangs-/Ausgangsbiegung für jeden Hilfskanal (6, 7) umfasst, wobei jeder Hilfskanal (6, 7) durch den Bund (3) bis in die Eingangs-/Ausgangsbiegung geht, die in die Eingangs-/Ausgangsöffnung (64, 74) mündet.

4. Kanüle (1) nach Anspruch 1 oder 2, wobei jeder Hilfskanal (6, 7) einen radial im Bund (3) ausgebildeten Abschnitt (63, 73) umfasst, wobei die Eingangs-/Ausgangsöffnung (64, 74) radial am Bund (3) angeordnet ist.

5. Kanüle (1) nach Anspruch 4, wobei jede radial am Bund (3) angeordnete Eingangs-/Ausgangsöffnung (64, 74) über den Abschnitt (63, 73) des Hilfskanals (6, 7), der sich radial im Bund (3) erstreckt, mit dem Hilfskanal (6, 7) verbunden ist und mit der von der Oberfläche des Bunds (3) definierten Ebene einen Winkel von zwischen 0° und 20° und insbesondere einen Winkel von ungefähr 10° bildet, um die entsprechende Eingangs-/Ausgangsöffnung (64, 74) zum Gesicht des Patienten auszurichten, wenn die Kanüle (1) an den Patienten angelegt ist.

6. Kanüle (1) nach einem der Ansprüche 1 bis 5, umfassend zwei flexible Injektions-/Extraktionsschläuche (8, 9), die hinter den Ohren des Patienten geführt werden können, wenn die Kanüle (1) an den Patienten angelegt ist, wobei ein erstes Ende jedes Schlauchs mit einer Eingangs-/Ausgangsöffnung (64, 74) verbunden ist.

7. Kanüle (1) nach Anspruch 6, umfassend einen Klemmring, der die beiden Schläuche (8, 9) zusammenhalten kann und entlang den beiden Schläuchen (8, 9) gleiten kann, um die Kanüle (1) an Ort und Stelle zu halten, wenn sie an den Patienten angelegt ist, so dass jeder Schlauch (8, 9) hinter einem Ohr verläuft.

8. Kanüle (1) nach Anspruch 6 oder 7, wobei ein zweites Ende der Injektions-/Extraktionsschläuche (8, 9) Mittel zur Verbindung mit Injektions- oder Extraktionsvorrichtungen umfasst.

9. Kanüle (1) nach einem der Ansprüche 1 bis 8, wobei der erste Hilfskanal (6) eine Eingangsöffnung (64) umfasst, die mit einem Schlauch (8) verbunden ist, um Sauerstoff in die Atemwege des Patienten zu injizieren, und der zweite Hilfskanal (7) eine Ausgangsöffnung (74) umfasst, die mit einem Schlauch (9) zur Extrahierung von Gasen, die vom Patienten ausgeatmet werden, verbunden ist, um eine kapnografische Messung durchzuführen.

10. Kanüle (1) nach einem der Ansprüche 1 bis 9, wobei sich der erste Hilfskanal (6) bis zu einem proximalen Teil (40) des röhrenförmigen Abschnitts erstreckt und sich der zweite Kanal (7) bis zu einem distalen Teil erstreckt, der das freie Ende (41) des röhrenförmigen Abschnitts (4) umfasst.

11. Kanüle (1) nach einem der Ansprüche 1 bis 10, wobei der erste Hilfskanal (6) in einer oberen Wand (S) des röhrenförmigen Abschnitts (4) des Körpers (2) ausgebildet ist und der zweite Hilfskanal (7) in einer unteren Wand (I) des röhrenförmigen Abschnitts (4) des Körpers (2) ausgebildet ist.

12. Kanüle (1) nach einem der Ansprüche 1 bis 11, wobei mindestens einer der Hilfskanäle (6, 7) mindestens am röhrenförmigen Abschnitt (4) des Körpers (2) einen länglichen Querschnitt umfasst.

13. Kanüle (1) nach einem der Ansprüche 1 bis 12, wobei mindestens einer der Hilfskanäle (6, 7) eine Öffnung umfasst, die in den Hauptkanal (5) mündet und eine kegelstumpfförmige Gestalt hat, deren Querschnitt größer als der mittlere Querschnitt des Hilfskanals (6, 7) ist.

14. Kanüle (1) nach einem der Ansprüche 1 bis 13, wobei das freie Ende (41) des röhrenförmigen Abschnitts (4) des Körpers (2) mit einer abgerundeten Gestalt versehen ist.

15. Kanüle (1) nach einem der Ansprüche 1 bis 14, wobei der Körper (2) aus flexiblem und glattem Kunststoff hergestellt ist.

## Claims

1. Nasopharyngeal cannula (1) comprising a body (2) formed by a tubular portion (4) that is curved so as to be inserted into a patient's nostril, and by a collar (3) that is able to rest on the base of the patient's nostril, the cannula (1) comprising a main conduit (5) forming a fluid passage between the collar (3) and the tubular portion (4) of the body (2), and two auxiliary conduits (6, 7) which are formed in the body (2) and extend from the collar (3) into the main conduit (5), **characterized in that** each auxiliary conduit (6, 7) opens into the collar (3) at an input/output orifice (64, 74) oriented radially with respect to the axis (II-II') of the main conduit (5) so as to be oriented laterally on each side of the patient's nostril when the cannula (1) is fitted on the patient.

2. Cannula (1) according to Claim 1, in which each input/output orifice (64, 74) is oriented radially with respect to the axis (II-II') of the main conduit (5) which forms an angle of between -10° and 20°, more particularly between 0° and 10°, with an axis orthogonal to the axis of the patient's nose when the cannula (1) is fitted on the patient, so as to orient the input/output orifices (64, 74) in the direction of the upper edge of the ears of the patient, when the cannula (1) is fitted on the patient.

3. Cannula (1) according to either of Claims 1 and 2, in which the collar (3) comprises an input/output bend for each auxiliary conduit (6, 7), each auxiliary conduit (6, 7) passing through the collar (3) into the input/output bend opening into the input/output orifice (64, 74).

4. Cannula (1) according to either of Claims 1 and 2, in which each auxiliary conduit (6, 7) comprises a portion (63, 73) formed radially in the collar (3), the input/output orifice (64, 74) being arranged radially on the collar (3).

5. Cannula (1) according to Claim 4, in which each input/output orifice (64, 74) arranged radially on the collar (3) is connected to an auxiliary conduit (6, 7) via the portion (63, 73) of the auxiliary conduit (6, 7) extending radially in the collar (3), forming an angle of between 0° and 20°, more particularly an angle of about 10°, with the plane defined by the surface of the collar (3), so as to orient the corresponding input/output orifice (64, 74) toward the patient's face, when the cannula (1) is fitted on the patient.

6. Cannula (1) according to one of Claims 1 to 5, comprising two flexible injection/extraction tubes (8, 9) which are able to be passed behind the patient's ears when the cannula (1) is fitted on the patient, a first end of each tube being connected to an input/output orifice (64, 74).

7. Cannula (1) according to Claim 6, comprising a clamping ring which is able to hold the two tubes (8, 9) together and can slide along the two tubes (8, 9) so as to hold the cannula (1) in place when it is fitted on the patient and each tube (8, 9) passes behind an ear.

8. Cannula (1) according to either of Claims 6 and 7, in which a second end of the injection/extraction tubes (8, 9) comprises means for connection to injection or extraction appliances.

9. Cannula (1) according to one of Claims 1 to 8, in which the first auxiliary conduit (6) comprises an input orifice (64) connected to an injection tube (8) for injecting dioxygen into the patient's airways, and the second auxiliary conduit (7) comprises an output orifice (74), which is connected to an extraction tube (9) for extracting gases exhaled by the patient so as to perform a capnographic measurement.

10. Cannula (1) according to one of Claims 1 to 9, in which the first auxiliary conduit (6) extends as far as a proximal part (40) of the tubular portion, and the second conduit (7) extends as far as a distal part comprising the free end (41) of the tubular portion (4).

11. Cannula (1) according to one of Claims 1 to 10, in which the first auxiliary conduit (6) is formed in an upper wall (S) of the tubular portion (4) of the body (2), and the second auxiliary conduit (7) is formed in a lower wall (I) of the tubular portion (4) of the body (2).

12. Cannula (1) according to one of Claims 1 to 11, in which at least one of the auxiliary conduits (6, 7) comprises a cross section of oblong shape, at least on the tubular portion (4) of the body (2).

13. Cannula (1) according to one of Claims 1 to 12, in which at least one of the auxiliary conduits (6, 7) comprises an orifice which opens into the main conduit (5) and has a frustoconical shape with a larger cross section than the mean cross section of the auxiliary conduit (6, 7).

14. Cannula (1) according to one of Claims 1 to 13, in which the free end (41) of the tubular portion (4) of the body (2) is provided with a rounded shape.

15. Cannula (1) according to one of Claims 1 to 14, in which the body (2) is made of flexible and sliding plastic.
